Europäisches Patentamt

European Patent Office

Office européen des brevets

(11) Publication number: **0 129 554**
**B1**

(12) **EUROPEAN PATENT SPECIFICATION**

(45) Date of publication of patent specification: **07.12.88**

(21) Application number: **83903732.2**

(22) Date of filing: **26.10.83**

(86) International application number:
**PCT/US83/01675**

(87) International publication number:
**WO 84/02473 05.07.84 Gazette 84/16**

(51) Int. Cl.⁴: **A 61 M 1/00, F 04 B 43/06**

(54) **PREPACKAGED FLUID PROCESSING MODULE HAVING PUMP AND VALVE ELEMENTS OPERABLE IN RESPONSE TO APPLIED PRESSURES.**

(30) Priority: **28.12.82 US 453926**
**28.12.82 US 453927**

(43) Date of publication of application:
**02.01.85 Bulletin 85/01**

(45) Publication of the grant of the patent:
**07.12.88 Bulletin 88/49**

(84) Designated Contracting States:
**BE CH DE FR GB LI SE**

(56) References cited:
**DE-A-2 723 197**
**GB-A-2 093 800**
**US-A-3 298 320**
**US-A-3 709 222**
**US-A-3 741 687**
**US-A-3 774 762**
**US-A-3 912 455**
**US-A-3 946 731**
**US-A-4 047 844**
**US-A-4 121 236**
**US-A-4 199 307**
**US-A-4 236 880**
**US-A-4 276 004**
**US-A-4 290 346**
**US-A-4 303 376**

(73) Proprietor: **BAXTER INTERNATIONAL INC.**
**One Baxter Parkway**
**Deerfield, IL 60015 (US)**

(72) Inventor: **BILSTAD, Arnold, C.**
**335 Pine**
**Deerfield, IL 60015 (US)**
Inventor: **BROWN, Richard, I.**
**2335 Peachtree Lane**
**Northbrook, IL 60062 (US)**
Inventor: **KRUGER, Robert, J.**
**1225 S. Patton**
**Arlington Heights, IL 60005 (US)**

(74) Representative: **MacGregor, Gordon et al**
**ERIC POTTER & CLARKSON 14 Oxford Street**
**Nottingham, NG1 5BP (GB)**

Courier Press, Leamington Spa, England.

## Description

Background of the Invention:

The present invention relates generally to fluid processing systems. More specifically, the present invention relates to a self-contained prepackaged fluid processing module which can be conveniently stored, set-up and operated. The module which embodies the features of the invention is ideally suited for medical procedures, such as those employed for blood fractionation and the like.

Various methods and apparatus have been developed which utilize disposable single-use processing systems formed of plastics such as vinyl for accomplishing fluid processing procedures. In the medical field, for example, processing systems have been developed for blood fractionation procedures, such as plasmapheresis, leukopheresis and plateletpheresis, wherein whole blood is separated into one or more fractions by means of either a filter element or by means of centrifugation, and for hemodialysis procedure, wherein diffusion exchange occurs through a membrane between whole blood and a dialysis solution.

In these blood fractionation procedures, it is typically necessary for an attendant to first select and locate an appropriate filter or membrane element and one or more flow sets. The packaging of these items must then be opened and the items must be connected together to form a fluid circuit, which is then installed on the particular processor apparatus with which the procedure is performed.

Typically, the processor apparatus includes multiple pump, detector and clamping elements on which particular components and tubing segments of the fluid circuit must be individually installed. Consequently, the set-up procedure may be undesirably complex so as to require a specially trained operator, and may require an undesirably long period to complete. Furthermore, even with the use of a specially trained technician, the potential remains for error, as where the wrong tubing segment is installed on a particular element of the apparatus.

Accordingly, the need has developed in the medical field for a modular blood fractionation system which contains in a single storable package all of the components required for a particular procedure, and wherein the connections between system components are clearly identified and preestablished so that the system can be quickly set-up and installed on an associated processor apparatus. Preferably, such a system and the associated apparatus should be constructed so as to avoid the need for installing individual tubing segments and components of the system on individual pump, monitor and clamp elements of the apparatus. Furthermore, such a blood fractionation system should contain all fluid containers necessary for fluids dispensed and collected in the procedure, so that the operator need only install the system in the actuator apparatus and connect input and output tubing segments to the donor prior to beginning a procedure.

US-A-3 774 762 discloses a fluid processing system for performing processing procedures for hemodialysis. The system includes a pair of superimposed flexible fluid-impermeable sheet members, one of which is supported to define a depressed portion so that a fluid passage is formed between the members. The sheets also define a pump chamber which can be repetitively compressed to effect a pump action. The two sheet members are sealed together and define a disposable component of the system. The precharacterising clause of Claim 1 is based on this disclosure.

The present invention is characterised in that the fluid processing system comprises a housing having a base, side walls upstanding from the base to define an open end and a space within the housing, and a removable cover closing the open end, the base defining the first member, the sheet member being accommodated in the housing and being more flexible than the base, and the fluid container being accommodated in said space.

The whole module may be disposable so that the principal components and interconnections required between them are contained within a single disposable housing.

The invention may best be understood by reference to the following description taken in conjunction with the accompanying drawings, in the several figures of which like reference numerals identify like elements, and in which:

Figure 1 is a perspective view of a blood fractionation module constructed in accordance with the invention for continuous-flow plasmapheresis, showing the cover thereof substantially removed to show the placement of the principal components of the system therein.

Figure 2 is a perspective view of the bottom of the blood fractionation module showing the fluid circuit formed on the bottom panel thereof.

Figure 3 is a cross-sectional view of the blood fractionation module taken along line 3—3 of Figure 2.

Figure 4 is a schematic diagram of the fluid circuit of the blood fractionation module.

Figure 5 is an exploded perspective view of the blood fractionation module showing the principal components and interconnections thereof.

Figure 6 is a perspective view of the blood fractionation module, disassembled and positioned for installation in the actuator station of an associated processor apparatus providing a continuous flow separation of plasma from whole blood.

Figure 7 is a perspective view of the blood fractionation module showing the module installed in the actuator station of the processor apparatus of Figure 6.

Figure 8 is a cross-sectional view of the blood fractionation module and the actuator station of the processor apparatus taken along line 8—8 of Figure 7.

Figure 9 is a front elevational view of the back plate provided in the actuator station for engaging the rear surface of the module housing.

Figure 10 is a front elevational view of the movable actuator head assembly provided in the actuator station of the processor apparatus.

Figure 11 is a side elevational view, partially cross-sectional and partially diagrammatic, of the processor apparatus showing the movable actuator head and actuating mechanism thereof, and the principal control circuits incorporated therein.

Figure 12 is a side elevational view, similar to Figure 11, showing the movable actuator head in a closed position engaging the blood fractionation module.

Figure 13 is a front elevational view of the bottom panel and overlying fluid-impervious flexible sheet member of the blood fractionation module partially fragmented to show the fluid circuit formed in the underlying bottom panel.

Figure 14 is a transverse cross-sectional view of the hollow-fiber filter element of the flow system taken along line 14—14 of Figure 13.

Figure 15 is a longitudinal cross-sectional view of the filter element taken along line 15—15 of Figure 13.

Figure 16 is a cross-sectional view of a connector fitting of the fluid circuit taken along line 16—16 of Figure 13.

Figure 17 is a cross-sectional view of the fluid absence detector incorporated in the fluid circuit taken along line 17—17 of Figure 13.

Figure 18 is a cross-sectional view similar to Figure 17 showing an alternative construction for the fluid absence detector.

Figures 19A—19C are enlarged cross-sectional views of a normally-closed pump element of the fluid circuit showing the element in at rest, fill and pump conditions, respectively.

Figures 20A—20C are enlarged cross-sectional views of a normally-open pump element of the fluid circuit showing the element in at rest, fill and pump conditions, respectively.

Figures 21A—21E are enlarged cross-sectional views of a bidirectional pump element of the fluid circuit showing the element in at rest, fill and pump conditions in one direction, and in fill and pump conditions in the other direction, respectively.

Figure 22 is a cross-sectional view of a pressure regulator element of the fluid circuit taken along line 22—22 of Figure 13.

Figure 23 is a cross-sectional view of a pressure sensor element of the fluid circuit taken along line 23—23 of Figure 13.

Figure 24 is a cross-sectional view of a hemolysis detector element of the fluid circuit taken along line 24—24 of Figure 13.

Description of the Preferred Embodiments

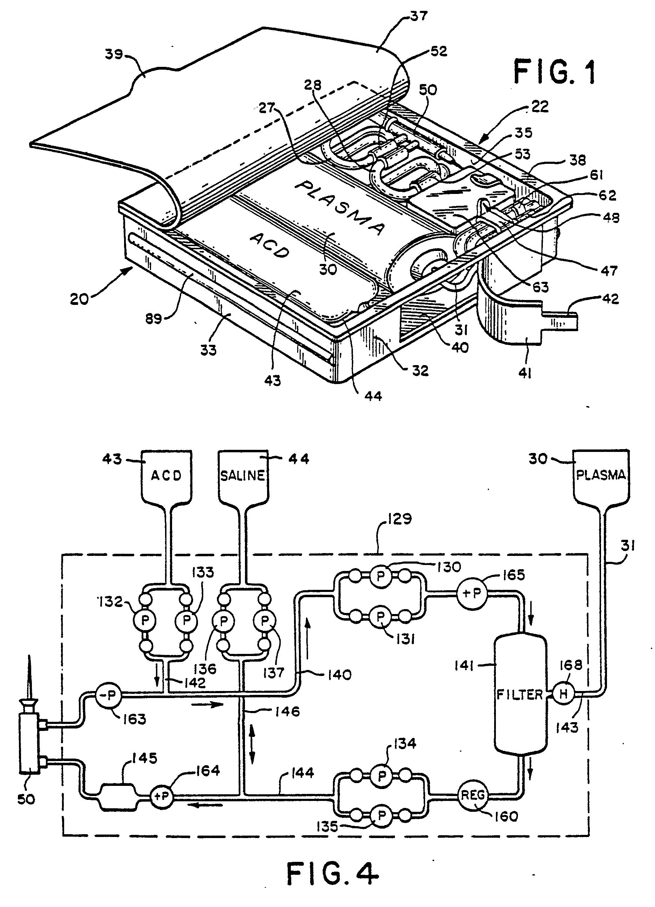

Referring to the drawings, and particularly to Figures 1—7, a prepackaged fluid processing module 20 is shown constructed in accordance with the invention for performing a fluid processing procedure in conjunction with an associated actuator apparatus 21. The module 20 includes a compact, normally closed housing 22 in which the fluid circuit and all the associated components required in the procedure are carried in a prearranged and, preferably, preattached condition. The housing 22 thus serves in part, as a compact storage container to protect the fluid circuit and components prior to use.

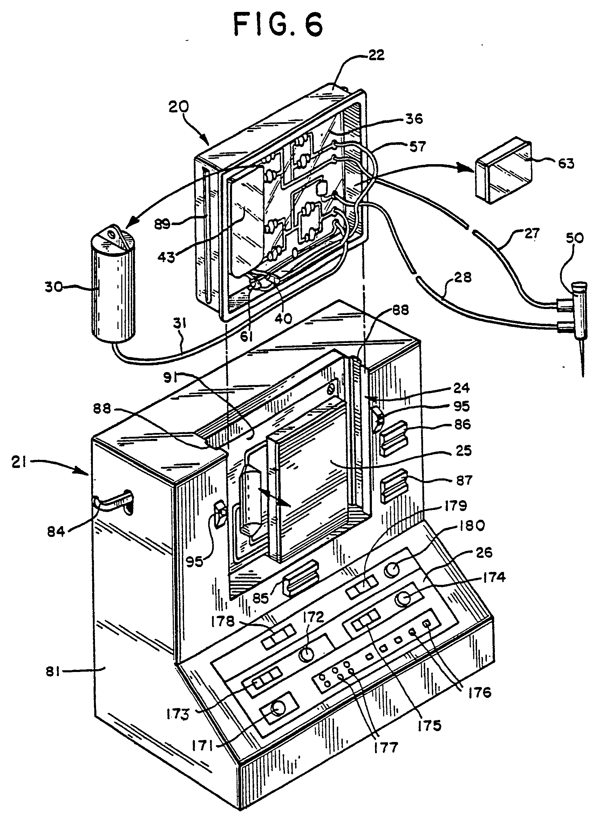

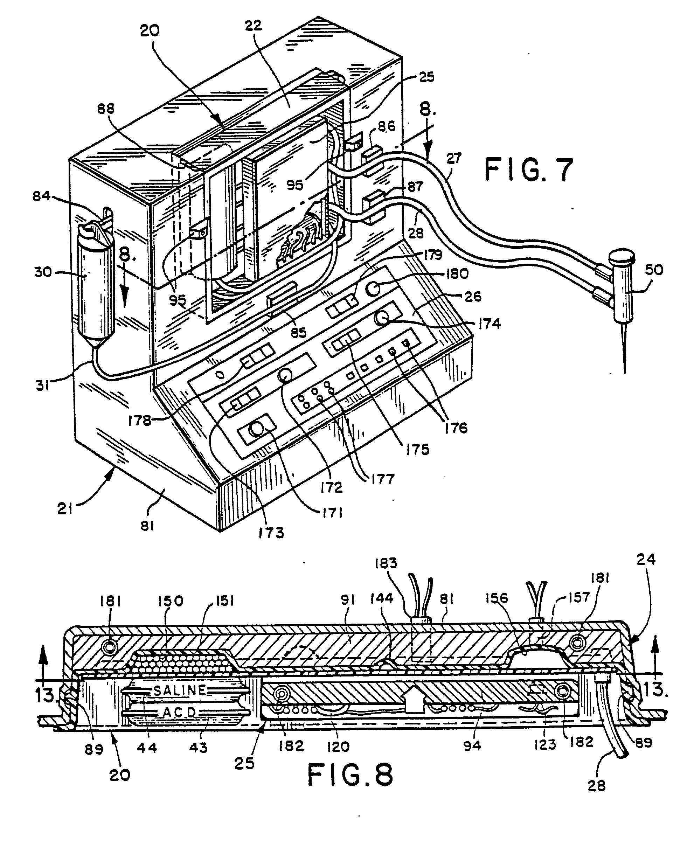

At the time of use, as shown in Figures 6 and 7, the housing can be opened and conveniently placed in operative association with an actuator station 24 of the actuator apparatus 21, which provides pumping and valving actions necessary in the fluid circuit of the module for accomplishing the plasmapheresis procedure. To this end, the actuator station may include an actuator head assembly 25 for engaging the housing, and a control panel 26 by which operating parameters for the procedure may be set by the operator.

The prepackaged, easily handled module 20, along with the associated actuator apparatus 21, are suited for use with virtually any fluid system. As will soon become apparent, the module 20 significantly simplifies the handling of fluid circuits, both prior to and during use, and all but eliminates the possibilities of an incorrectly arranged flow system or an incorrectly connected pump.

The inherent advantages of the modular flow system of the invention particularly lend the invention to use in fluid processing systems in the medical field, and particularly for fractionating or otherwise processing blood. For this reason, use of the module 20 in this context will be described.

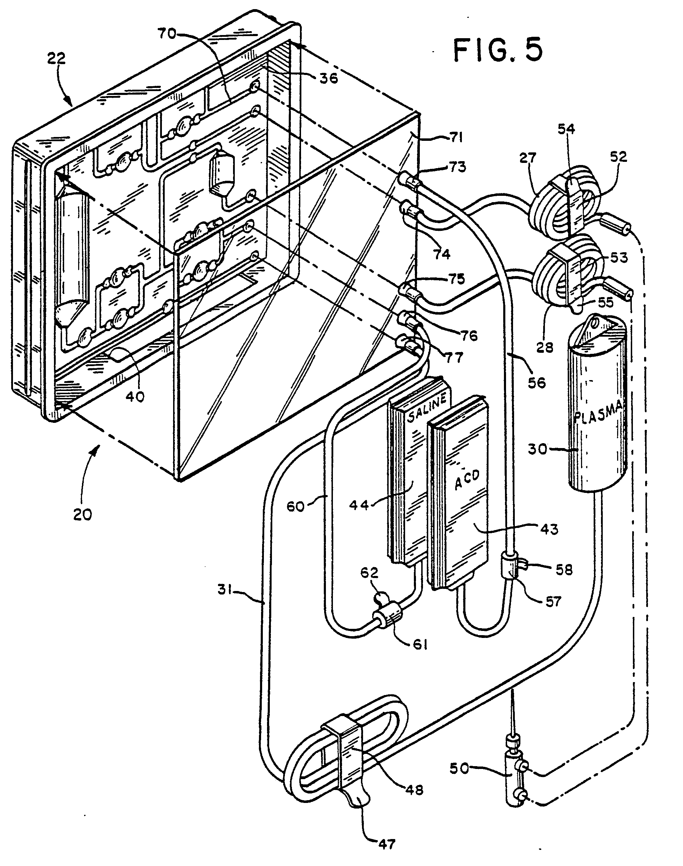

While the specific blood processing procedure performed by the module 20 may vary, in the illustrated embodiment a fluid circuit 29 (Figure 4) for performing a continuous flow membrane plasmapheresis procedure is shown. During this plasmapheresis procedure, whole blood is drawn from a donor through a tubing segment 27 and separated into a plasma component and a red blood cell component. The red blood cell component is returned to the donor through a tubing segment 28. The plasma component is delivered to a container 30 through a tubing segment 31 for fractionation into various therapeutic components, such as albumin or Clotting Factor VIII.

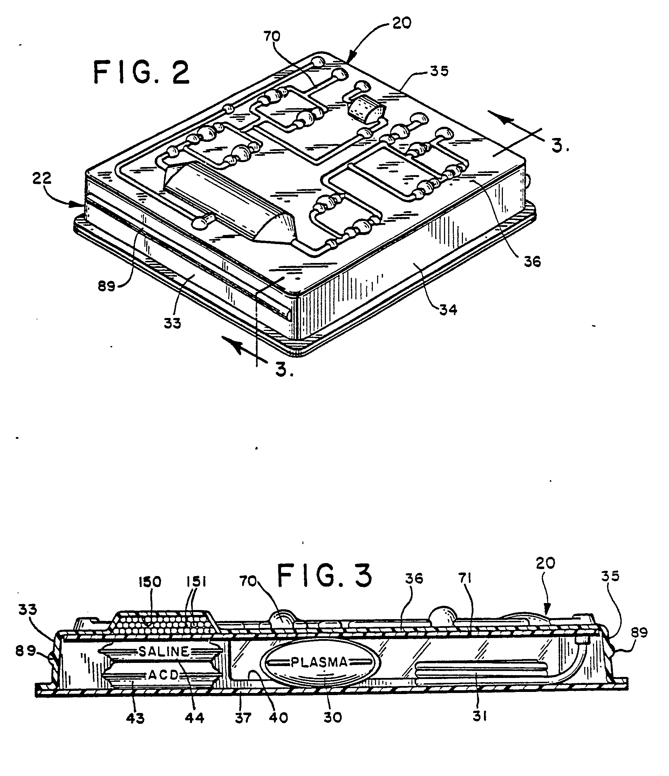

Referring now to Figures 1—5, the housing 22 is seen to comprise four side panels 32—35 and a bottom panel 36 formed of a relatively rigid, fluid-impervious material, preferably translucent, clear or colored, and not opaque to light, such as molded plastic. The housing 22 is normally closed and sealed for long term storage by means of a cover 37, which is sealed over the open end of the housing. The cover may be formed of a flexible, fluid-impermeable material such as metallic foil or vinyl, and may be secured by means of a layer 38 of adhesive or other appropriate means to the edge of the housing, as shown in Figure 2. The housing side panels 32—35 preferably include an outwardly projecting rim portion to facilitate this attachment. A pull tab 39 may be provided to assist the operator in removing the cover at time of use.

To facilitate the desired operative interface between the module 20 and the actuator station 24 of processor apparatus 21, an elongated aperture 40 (see, in particular, Fig. 1) is provided in side panel 32. This aperture 40 is normally closed and sealed during storage by means of a cover 41, which extends over the aperture and is secured to side panel 32 by a layer of adhesive or other appropriate means. A pull tab 42 may be provided to facilitate removal of cover 41 from side panel 32 when preparing the processing system for use.

The fluid circuit and all the components required to perform the particular desired procedure can be conveniently carried within the confines of the housing 22.

In the context of a continuous flow plasmapheresis procedure, containers are required for storing an anticoagulant solution (such as ACD or CPDA), a saline solution, and the collected plasma. Accordingly, the fluid processing system located within the housing in the illustrated embodiment includes a prefilled ACD container 43, a prefilled saline container 44 and the empty plasma collection container 30.

The prefilled ACD container 43 and saline container 44 are preferably formed of flexible vinyl material. To prevent the contents of the containers 43 and 44 from evaporating through the vinyl walls of the containers, a suitable over-wrap (see Fig. 5) is preferably provided to form a vapor barrier around the containers.

The containers 43 and 44 may be stored within housing 22 in an unconnected condition with the associated fluid circuit. In this arrangement, at the time of use, containers 43 and 44 are removed from the housing 22, the overwraps are removed, and the containers are then connected by the operator in flow communication with the circuit. In making the connection in an open or aseptic system, a conventional blood "spike" carried on tubing connected to the fluid circuit can be used to pierce a membrane associated with the inlet port of the end container. Alternately, in making the connection in a closed or sterile system, a sterile connector device can be used to interconnect the containers 43 and 44 with the fluid system, such as disclosed in US-A-4,157,723.

Preferably, the containers 43 and 44 are stored within the housing in a preattached condition with the fluid circuit. The containers 43 and 44, along with the associated overwraps may be integrally connected with the circuit using a port block. In this arrangement, the containers 43 and 44 are preferably permanently secured within housing 22 by adhesive attachment to the interior surface of the adjacent side panel 34.

When in a preattached condition, the ACD container 43 is integrally connected to the fluid circuit of the processing system by a tubing segment 56, which includes a frangible in-line cannula 57 (see Fig. 5), such as disclosed in US-A-4,294,247. This cannula 57 is preferably arranged so as to be readily accessible to the operator upon removing cover 37, and preferably also includes a pull tab 58 containing indicia and/or color coding to draw the operator's attention to the cannula 57 during the set-up procedure.

Similarly, when preattached, the saline container 44 is integrally connected to the fluid circuit by means of a tubing segment 60 which includes another frangible in-line cannula 61. This cannula 61 is also preferably positioned for ready access to the operator upon removing cover 37, and also includes a pull tab 62 containing indicia and/or color coding to assist the operator in locating and fracturing the cannula during set-up.

Plasma container 30, which may be in the form of an unbreakable bottle of the type which conforms to standards established for long term plasma storage, is preferably not permanently secured and can be removed by the system operator at time of use. As is best shown in Fig. 5, tubing segment 31 is preferably prearranged in a compact bundle in housing 22 and secured by an adhesive strip 47 or other appropriate means to an adjacent side panel 32 (see Fig. 1) so as to be readily accessible when preparing the processing system for use. A pull tab 48 bearing appropriate indicia and/or color coding may be provided at one end of adhesive strip 47 to facilitate removing the strip from the tubing bundle.

Tubing segments 27 and 28 are each connected at one end to the processing circuit, and at their other end to respective ports of a conventional dual lumen phlebotomy needle 50 to accommodate a "single needle" plasmapheresis procedure. Alternately, each tubing segment 27 and 28 could individually communicate with a separate phlebotomy needle to accommodate a "two needle" plasmapheresis procedure.

Tubing segments 27 and 28 are also each preferably prearranged in a compact coil within housing 22 and secured by respective adhesive strips 52 and 53 in this form. Pull tabs 54 and 55 identified by appropriate indicia and/or color coding may be provided to assist the operator in locating and removing the adhesive strips from these tubing segment coils.

As shown in Figure 3, the ACD and saline containers 43 and 44 can be generally wide and relatively flat so as to accommodate their compact stacking one-above-the-other within the housing 22. The plasma container 30, which, as previously described, is preferably in the form of a standard plasma collection bottle, can then be conveniently positioned to one side of the stacked saline and ACD containers 43 and 44 so as to allow room for tubing coils 27, 28, and 31.

Additional ancillary components and items required during the plasmapheresis process may be contained within a small rectangular miscellaneous supplies container 63, disposed within housing 22 (see Fig. 1). This container 63 may include hypodermic needles, bandages, surgical tape, antiseptic solution, and other items incidental to the procedure.

As shown in Figures 2 and 5, the bottom panel 36 of the housing 21 is molded or vacuum formed to include portions 70 which project or bow outwardly from the housing interior. Together, these portions 70 define in the interior surface of the bottom panel the fluid flow pattern required to perform the plasmapheresis procedure. This preformed fluid circuit also includes a plurality of chambers which provide necessary valving, pumping, filtering and fluid detector capabilities when the system is installed in actuator station 24.

As is best shown in Fig. 5, the preformed fluid circuit defined in the interior portion of bottom panel 36 is sealed by means of a fluid-impermeable flexible plastic sheet member 71 which fits over the interior surface of the bottom panel 36. This flexible sheet member 71 is preferably dimensioned to correspond to the inside peripheral dimensions of bottom panel 36 and is secured over the panel 36 by RF welding, or other appropriate means. Together, the portions 70 and the overlying flexible sheet member 71 form the entire fluid-sealed fluid circuit within the module.

Fluid communication is selectively established with the fluid circuit by means of five connector fittings 73—77 which extend through respective apertures in flexible sheet member 71. When sheet 71 is sealed in position against the inside surface of bottom panel 36, the connector fittings align and engage respective ones of five inlet/ outlet port locations preformed on the interior surface of the back panel 36. Tubing segment 56 is connected to connector 73 at one end and to the ACD container 43 at its other end. Tubing segment 27 is connected at one end to connector 74 and at its other end to the needle 50. Tubing segment 28 is connected to connector 75 at one end and to the needle 50 at its other end. Tubing segment 60 is connected between connector 76 and saline container 44. Tubing segment 31 is connected between connector 77 and plasma collection container 30. Fluid flow into and out of the fluid circuit is thus provided.

By virtue of this above-described construction, each of the components contained within housing 22 is interconnected in a preattached condition for storage and eventual use in a plasmapheresis procedure. No additional interconnections need be made by the operator during set-up.

Referring to Figure 6, prior to use of the processing module 20, the cover 37 is removed and the plasma collection container 30, tubing segments 27, 28 and 31, and the accessories container 63 are removed from housing 22. The cover 41 is then removed, and the system is installed in the actuator station 24 of processor apparatus 21, which provides the fluid pumping, valving and monitoring functions necessary in accomplishing the plasmapheresis procedure. Significantly, and in accordance with another aspect of the invention, all of these functions are accomplished without the necessity of individually installing tubing segments and other components of the system in separate pumping, valving and sensing elements of the apparatus. Instead, upon installation of the module 20 in the actuator station 24 of the processor apparatus, all such functions are realized automatically without individual attention by the operator and without interrupting the integrity of the fluid circuit of the processing system.

Fluid pumping and valving functions are achieved by application of pressure forces to the flexible sheet member 71 which overlies the bottom panel 36 of housing 22. To this end, actuator apparatus 21 includes a housing 81 having a generally vertical portion within which the actuator station 24 is provided for receiving housing 22, and a lower base portion on which control panel 26 is provided. A hanger arm 84 is provided on the left (as viewed in Figure 6) side of the upper housing portion to support the plasma collection container 30, and three tubing retention blocks 85—87 are provided for supporting respective ones of tubing segments 31, 27 and 28 when housing 22 is seated in station 24.

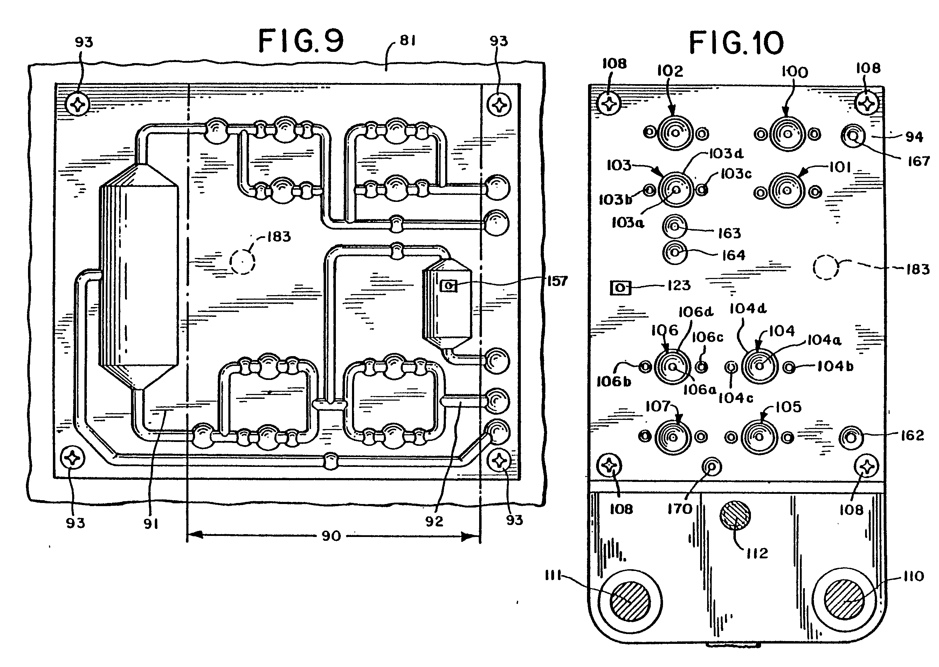

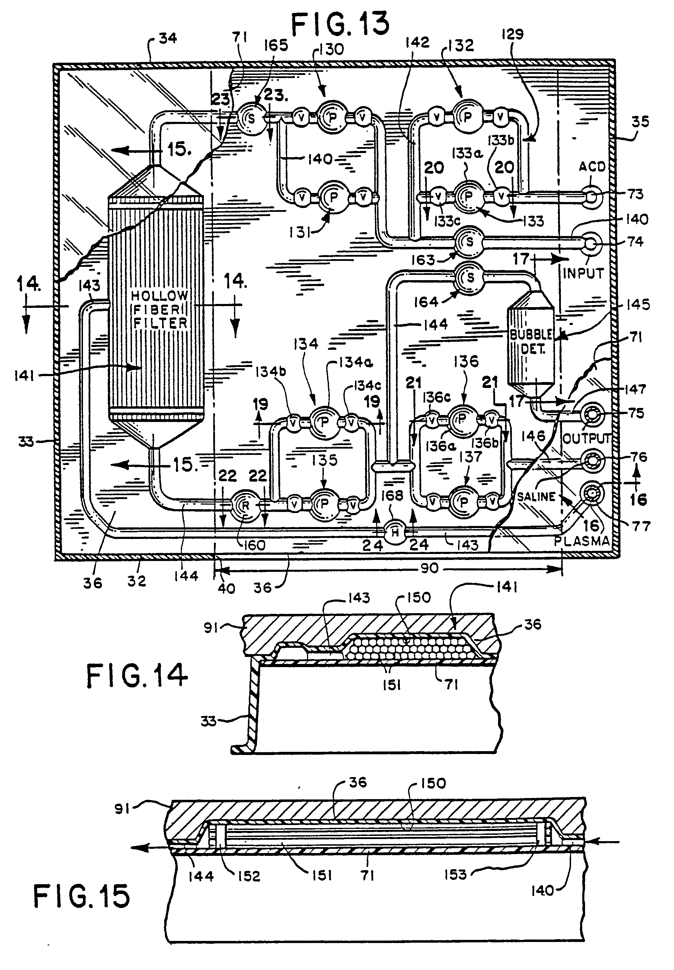

Actuator station 24, which is dimensioned to receive housing 22 in sliding engagement, may include a pair of slots 88 for engaging ribs 89 molded into the side of the housing to maintain the housing in accurate alignment. In operation, pressure communication with the processing system is established by means of the movable actuator head 25 within actuator station 24. When housing 22 is installed in station 24, the actuator head extends through aperture 40 in side panel 32 so as to overlie a portion 90 (Figures 9 and 13) of sheet member 71 and the underlying portion of bottom panel 36. To enable pressure forces to be applied to the flexible sheet member 71, the actuator head is brought into engagement with the sheet member by reciprocative movement toward the rear of actuator station 24. This causes the bottom panel and sheet member to be compressed between the head and a back plate 91 at the rear of the station.

As shown in Figure 9, the back plate 91 may include a series of depressions, collectively identified as 92, arranged to receive the raised portions 70 of bottom panel 36. The back plate 91 may be secured to the housing 81 of processor 21 by means of a plurality of machine screws 93, or other appropriate fastening means. By removing back plate 91 and substituting a different back plate having a different pattern of depressions 92, apparatus 21 can be reconfigured for other fluid processing procedures. A pair of retaining clamps 95 may be provided for holding the module 20 in actuator station 24.

As shown in Figures 8—12, the actuator head assembly 25 includes an actuator plate 94 which is brought into engagement with sheet member 71. As shown in Figure 10, this actuator plate includes eight pneumatic pump actuator elements 100—107 which apply a vacuum and/or pressure control effect to appropriate portions of sheet member 71 associated with corresponding pump elements in the fluid circuit defined by the sheet member and bottom panel 36 to obtain necessary pumping and valving functions. The actuator plate 94 is preferably mounted within actuator head assembly 25 by means of a plurality of machine screws 108, or other appropriate fastening means, so that the actuator plate can be removed and a different actuator plate having a different arrangement of vacuum actuator ports can be substituted when reconfiguring the processor apparatus for a different fluid processing procedure.

Referring to Figures 10—12, the actuator head 25 is mounted on a pair of parallel-spaced guide rods 110 and 111 for reciprocative movement toward and away from back plate 91. The head assembly is positioned along the guide rods by means of a jackscrew 112, drive gear assembly 113 and motor 114. Upon rotation of motor 114, the jackscrew 112 is turned and the actuator head is caused to move. An actuator head control circuit 115 functions in conjunction with limit switches 116 and 117 to limit actuator head travel between open and closed positions.

Within actuator head 25 the individual pneumatic actuator elements 100—107 are connected by respective tubing segments collectively identified as 120 to actuator circuits 121. These actuator circuits respond to electrical command signals issued by a processor control circuit 122 within processor apparatus 21 to selectively apply either a vacuum or pressure differential at the actuator ports, as required in performing the fluid processing procedure. Also, one or more sensing elements such as an ultrasonic detector 123 may be provided in the actuator head assembly to sense the occurrence of a fluid absence in the fluid circuit. This detector may be connected by a conductor 124 to appropriate sensing circuits 125 within the apparatus, which provide an appropriate output signal to control circuits 122 upon the occurrence of a fluid absence. In addition, control circuits 122 may receive operator-initiated command signals from control panel 26.

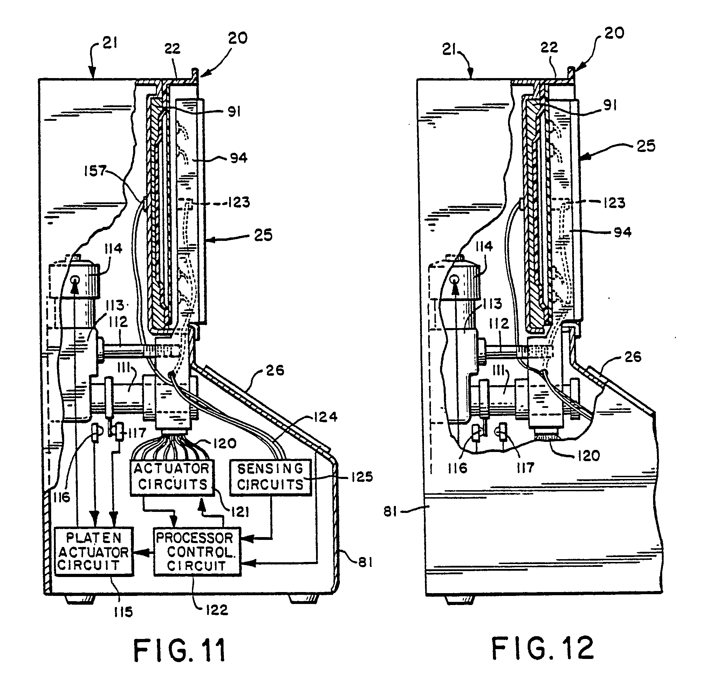

In the open position of actuator head 25, as shown in Figure 11, the housing 22 of the processing system is received with the bottom panel 36 thereof interposed between actuator plate 94 and back plate 91. Then, upon issuance of an appropriate operator-initiated signal on control panel 26, control circuits 122 condition platen actuator circuit 115 to cause motor 114 to close the actuator head. This brings the pressure actuator ports of actuator plate 94 into tight engagement with back panel 36, as shown in Figure 12. Upon completion of the procedure, the process is reversed to enable housing 22 to be removed and the processing system to be disposed of.

The fluid circuit formed between the bottom panel 36 of housing 22 and the overlying fluid-impermeable sheet member 71, which is collectively identified by the number 129 in Figure 13, includes eight preformed pump elements 130—137 for establishing flow through the fluid circuit. These pump elements, which are actuated by respective ones of actuator elements 100—108 in actuator plate 94, operate in pairs, each pair member being alternately actuated to establish a continuous flow of fluid. Pump elements 130 and 131 are paired to pump whole blood, as received from a donor through tubing segment 27 and connector 74, through a conduit segment 140 to a filter element 141. Pump elements 132 and 133 are paired to pump anticoagulant (ACD) fluid from tubing segment 56 and connector 73 through a conduit segment 142 into conduit segment 140, wherein it is combined with whole blood. Within filter element 141 plasma is separated, and separately supplied to port 77 through a conduit segment 143. Pump components 134 and 135 pump plasma-deficient whole blood from filter element 141 through a conduit segment 144 to a fluid absence detector element 145. Pump elements 136 and 137 operate to combine saline from tubing segment 60 and connector 76 through a conduit segment 146 to the plasma-deficient blood in conduit segment 144. Plasma-deficient blood from fluid absence detector element 145 is supplied through a conduit segment 147 to connector 75 and tubing segment 28 for return to the donor.

Referring to Figures 14 and 15, the plasma filter element 141 comprises a chamber 150 preformed within bottom panel 36 within which a plurality of hollow fiber filter elements 151 are arranged side-by-side in a longitudinal bundle to convey fluid received from conduit segment 140 to conduit segment 144. Adjacent each end of the filter bundle layers 152 and 153 of a fluid-impermeable material such as polyurethane are deposited to form liquid barriers which prevent fluid flow except through the hollow fibers of the filter. Plasma collected in the space surrounding the hollow fiber filter elements is conveyed through conduit segment 143 to connector 77 and tubing segment 31 for ultimate collection in the plasma collection container 30. While a hollow-fiber type filter membrane is shown, it will be appreciated that other forms of filter membranes, such as a flat sheet, may be provided within chamber 150 instead.

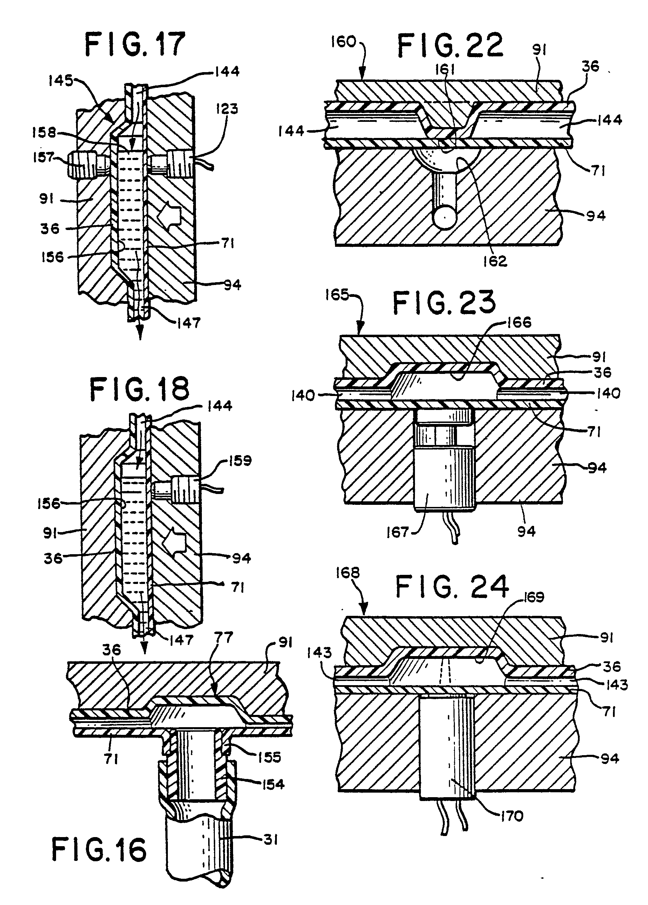

The connector element 77, which is identical in construction to connector elements 73—76, is seen in Figure 16 to comprise a short conduit segment 154 having an outside diameter corresponding to the inside diameter of tubing segment 31. The tubing segment is forced over one end of the conduit segment, and the other end is received within a projecting flange portion 155 of the flexible sheet member 71. A bonding material is applied between the ends of the conduit segment and the sheet member and tubing segment to provide a durable fluid seal.

Referring to Figure 17, the fluid absence detector 145 comprises a vertically-orientated chamber 156 through which plasma-deficient whole blood flows prior to being reinfused into the patient. When processing module 20 is installed in actuator 21 chamber 156 is vertically aligned, so that should a fluid absence develop, as a result of a pump malfunction, an occlusion, or any other reason, a fluid void will develop at the upper end of the chamber. A fluid detector system comprising the ultrasonic transmitter 123 on actuator plate 94 and a receiver 157 on back plate 91 sense the presence or absence of whole blood at a predetermined level 158 within chamber 156. Upon the detector sensing a fluid absence at this location, operation of the plasmapheresis processing system is terminated and an alarm is sounded, in accordance with conventional practice. The fluid detector, and its associated circuitry may be similar to that described in US-A-4,341,116.

An alternative construction for the ultrasonic fluid absence detector is shown in Figure 18. In this construction a combined transmitter-receiver transducer element 159 is situated on the movable actuator plate 94. The transmitter portion of the transducer introduces ultrasonic energy into chamber 156 and the detector portion of the transducer responds to reflected energy to produce an output signal indicative of the presence or absence of fluid in the chamber. In this way the detector system does not rely on the transparency of the bottom panel 36 of housing 22, thereby avoiding the possibility of the detector being rendered inoperative by tape or other material adhering to the outside surface of the bottom panel.

As shown in Figure 13, each of the eight pump elements 130—137 in fluid circuit 129 includes a central displacement chamber (a), an upline valve stop (b), and a downline valve stop (c). As shown in Figure 10, the eight pump actuator elements 100—107 on actuator plate 94 each include a central pumping port (a), an upline valving port (b), and a downline valving port (c), which interact with the central displacement chamber, upline valve stop, and downline valve stop of their counterpart pump elements in the fluid circuit. In addition, each of the eight pump actuator elements includes an annular hold-down port (d) which encircles the pump actuator port (a).

The operation of pump element 134 is illustrated in Figures 19A—19C. As shown in the Figures, the inlet valve consists of an actuator port 104b formed in the actuator plate 94 of the movable actuator head assembly 25, and a valve stop 134b formed in the rigid bottom panel 36 of housing 22. Similarly, the outlet valve consists of an actuator port 104c and a valve stop 134c. Each pump chamber is formed by a pump port 104a in the actuator plate and a displacement chamber 134a in the bottom panel.

At rest, as shown in Figure 19A, processor apparatus 21 provides no vacuum at either the upline valving chamber 104b or the downline valving chamber 104c. Consequently, sheet member 71 rests in contact with the inlet and outlet valve seats 134b and 134c. This condition may be enhanced by providing a positive pressure at valving chambers 104b and 104c.

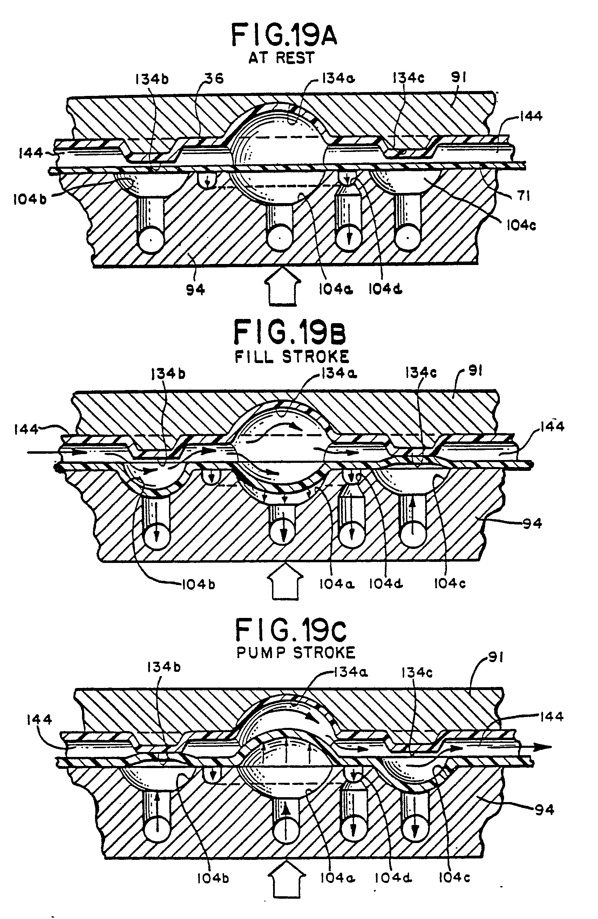

During operation of the module, a pump fill stroke is initiated by processor apparatus 21 by drawing a vacuum in inlet valving chamber 104b to draw sheet member 71 into the valving chamber. This opens the inlet valve and allows fluid to flow through the valving chamber and into the displacement chamber 134a. At this time a vacuum is slowly drawn at the pumping port 104a so as to draw the sheet member 71 to the bottom (as viewed in Figure 19B) of the displacement chamber to cause fluid to enter the chamber. At this time the downline valve is closed by reason of a positive pressure being applied at valving port 104c, causing sheet member 71 to be displaced against the valve stop 134c.

Upon completion of the fill stroke a pump stroke is initiated by processor apparatus 21. Inlet valving port 104b is pressurized to displace sheet member 71 against valving shoulder 134b. At the same time, a vacuum is drawn at outlet valving port 104c to draw sheet member 71 into the valving port thereby opening the outlet valve. A positive pressure is next slowly introduced into pumping port 104a to force sheet member 71 upwardly (as viewed in Figure 19C) to displace fluid within the fluid displacement chamber 134a.

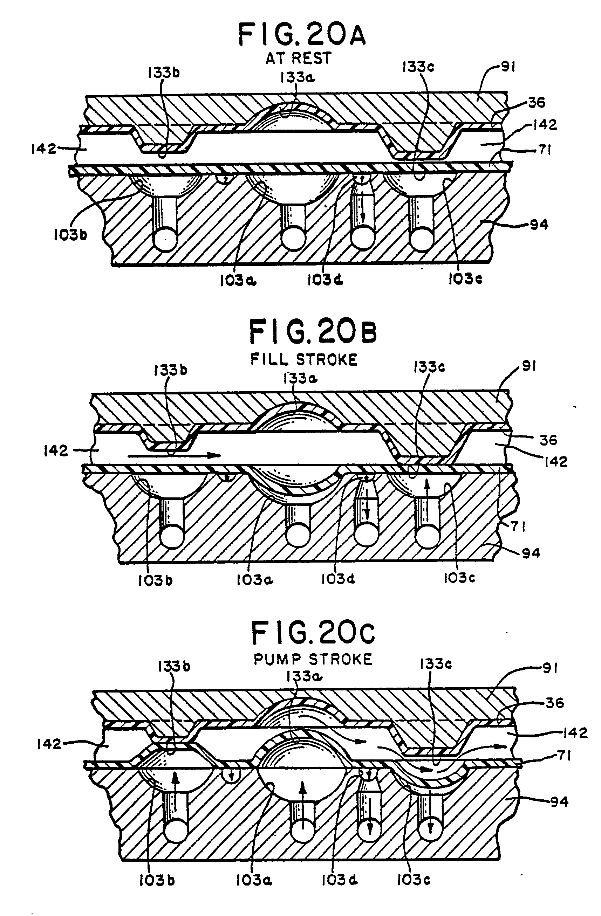

An alternative construction for the pump elements is shown in Figures 20A-20C, which depict the construction and operation of the ACD pump element 133. Instead of a normally closed inlet valve stop 134b provided in pump element 134, valve 133 utilizes a normally open valve stop 133b which is closed only during the pump stroke upon application of positive pressure to the inlet valve control port 103b, as shown in Figure 20c. By avoiding the need to draw a vacuum in inlet valve control port 103b during rest and fill strokes valve element 133 conserves energy within the apparatus and simplifies the associated valve actuator apparatus.

The pump chamber 133a and outlet valve 133c of valve 133 are identical in construction and operation to those of valve 134. Outlet valve 133c is closed by positive pressure during the fill stroke, and opened by negative pressure and/or fluid pressure during the pump stroke. The pump element actuator ports 103a-103d are identical to those of valve 134.

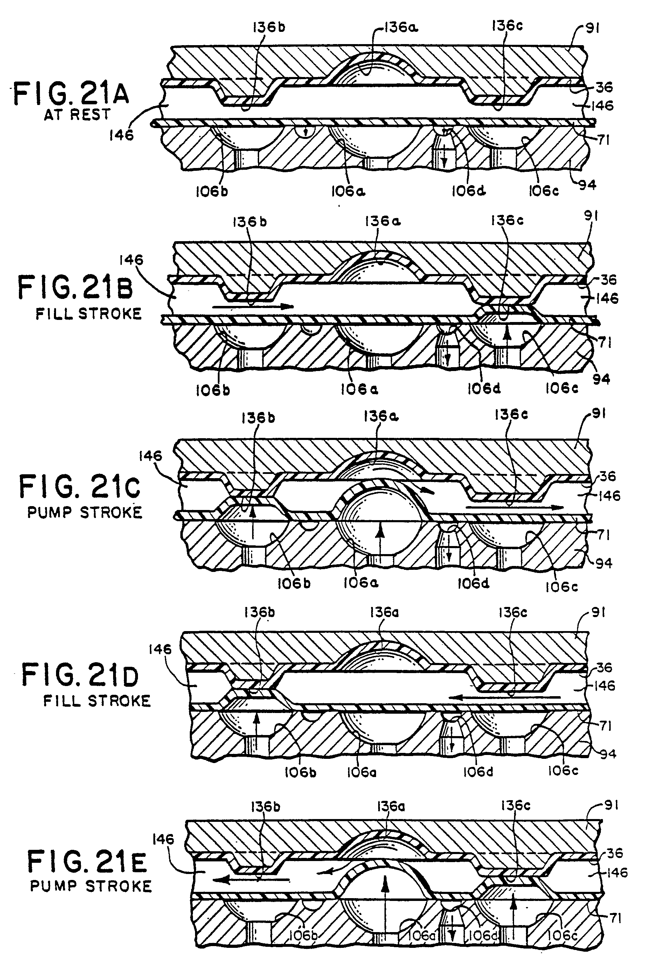

Another alternative construction for the pump elements is shown in Figures 21A—21E, which depict the construction and operation of the bidirectional saline pump element 136. This pump element utilizes two normally or partially open valve elements 136b and 136c, which can be actuated by appropriate pressures through pressure ports 106b and 106c respectively. As shown in Figure 20A, at rest both valves are open and fluid can flow through the pump element.

For left-to-right flow, as illustrated in Figure 21B and 21C, valve 136b is open during each fill stroke, and closed by positive pressure at port 106b during each pump stroke. At the same time, valve 136c is closed by pressure applied through port 106c during each fill stroke, and allowed to remain open during each pump stroke. For right-to-left flow, as illustrated in Figures 21D and 21E, valve 136b is closed during each fill stroke, and valve 136c is closed during each pump stroke.

Thus, valve element 136 is able to pump fluid in either direction, with a minimal pressure requirement for valve actuation. This makes the valve construction well suited for use in the saline conduit 146, where flow may take place into the system during normal replacement procedures, and out of the system during prime and purge procedures.

For optimum efficiency in separating plasma it is desirable that the transmembrane pressure (TMP) present in the hollow-fiber filter element 141 be controlled and regulated. To this end, the fluid circuit 129 includes in-line in conduit segment 144 a pressure regulator element 160. Referring to Figure 22, this regulator comprises a valve stop 161 in the rigid bottom panel 36 and an underlying pressure port 162 in actuator plate 94. With this arrangement it is necessary that the flexible sheet member 71 be deflected downwardly into pressure port 162 and away from valve stop 161 before fluid can flow through conduit 144. Pressure regulation is provided by introducing a predetermined metering pressure in chamber 162 in opposition to deflection of sheet member 71, so that the valve opens only when the desired pressure level has been reached in conduit 144, and modulates to maintain the desired pressure once flow has been initiated. By maintaining the metering pressure constant, the desired upline pressure is maintained in conduit 144 and filter element 141.

In some applications it may be possible to avoid the need for pressure regulator element 160 by appropriately biasing the normally-closed inlet valve chambers 134b and 135b so that pump elements 134 and 135 function as pressure regulating elements. The outlet valves 134c and 135c would then remain unbiased by pneumatic pressure so as to open in response to applied fluid pressure upon the opening of inlet valves 134b and 135c.

To permit the monitoring of system operating pressures, fluid circuit 129 includes three pressure monitor elements 163—165.

Pressure monitoring element 163 is located in conduit segment 140 and monitors negative pressure to detect the occurrence of a collapsed vein. Monitor 164 is located in conduit segment 144 and monitors positive pressure to detect the occurrence of an occlusion in the output circuit. Monitor 165 is located in the input conduit 140 to the filter element 141 to monitor filter inlet pressure, and hence TMP.

Referring to Figure 23, filter element monitor 165, which may be identical to elements 163 and 164, is seen to comprise an in-line chamber 166 formed in conduit segment 140, and a conventional pressure transducer 167 mounted within actuator plate 94 and positioned to operatively engage the flexible sheet member as it overlies the chamber. Pressure variations in the fluid, which necessarily exist in the chamber, are reflected in changes in the electrical output signal produced by transducer 167. These signals are utilized by appropriate control circuitry in actuator apparatus 21 to provide readouts of system parameters and to control the operation of the fluid circuit.

To prevent red blood cells from being inadvertently collected in plasma collection container 30, the fluid circuit 129 includes a hemolysis detector 168 in conduit segment 143. Basically, this detector includes an in-line chamber 169 through which collected plasma is caused to flow. The presence of red blood cells in this chamber is detected by a detector 170, which may include two monchromatic light sources of different wavelengths, a light detector responsive to reflection of light from within the chamber at the two wavelengths, and circuitry responsive to the light detector for providing an alarm. The detector system may be as described in US-A-4,305,659.

To maintain a continuous non-pulsating flow through the fluid circuit, the paired fluid pump elements are operated in alternation. That is, when one pump component is operated in a fill stroke, its pair is operating in a pump stroke. In this way, an uninterrupted non-pulsating flow of fluid is maintained through the fluid circuit.

Specifically, elements 132 and 133 are paired and operated to introduce ACD into the main fluid conduit 140 at an operator-designated rate to prevent blood clotting. Pump elements 130 and 131 advance the whole blood obtained from the donor together with the ACD to the hollow fiber filter element 141. Within this element the whole blood is fractionated and the derived plasma component is discharged through conduit segment 143 to the plasma collection container 30.

The plasma-deficient output from filter 141 is advanced along conduit segment 144 by pump elements 134 and 135, which operate in alternation to maintain a smooth non-pulsating flow at this point. Pump elements 136 and 137 introduce saline into the main flow conduit 144 at an operator-designated rate as a plasma replacement fluid, or pump fluid and/or trapped air into saline container 44 during prime and purge procedures.

By controlling the rate at which pressure differentials are established in the pump actuator ports 100a—107a a gentle and natural pumping action is obtained which provides minimal damage to processed blood cells. The pumping rates of the individual pump elements may be set by the operator, or by automatic means within the processor apparatus responsive to measured system parameters, such as the volume and rate of plasma collection. To this end, control panel 26 (Figures 6 and 7) of apparatus 21 includes a selector switch 171 by which the operating speed of the anticoagulant pump element is set, a potentiometer control 172 and digital readout 173 by which the operating speed of whole blood pump elements 130, 131 and 134, 135 is controlled, and a potentiometer 174 and digital readout 175 by which the operating speed of the replacement fluid pump element 136, 137 is controlled. A plurality of push button switches 176 are provided to establish the operating mode of the apparatus, and a plurality of status-indicating lights 177 provide indications of malfunctions in the system. A digital readout 178 indicates the total volume of plasma collected, and a digital readout 179 indicates plasma collection rate. A selector switch 180 enables the replacement fluid rate to be set as a ratio of the actual plasma collection rate.

Since the displacement chambers 130a—137a of the pump elements 130—137 have a fixed and constant volume, fluid flow within the fluid circuit 129 can be controlled with great accuracy by controlling the number of pump actuations. Since each pump pulse may be treated as an aliquot, processing, proportioning and timing operations are easily accomplished in the course of the procedure.

In many procedures, such as continuous flow blood fractionation, or hemodialysis, it is desirable that the blood being processed in module 20 be maintained at a constant predetermined temperture, such as 98°F (37°C). To this end, processor apparatus 21 may, as shown in Figure 8 include a resistance heating element 181 in back plate 91, and a resistance heating element 182 in actuator plate 94, in addition to appropriate thermal insulation for these components. These elements are powered by suitable circuitry within the processor apparatus in accordance with the temperature sensed by a temperature sensing element 183 on back plate 91 to maintain the desired temperature. Because of the minimal blood volume in process at any one time, and the intimate contact between the blood circuit 129 and the relatively massive actuator plate 94 and back plate 91, efficient thermal transfer is realized and fluid temperature accurately maintained.

Alternatively, where it is desired that the blood in process be cooled, as in cryoagulation procedures, or in the secondary treatment of plasma, cooling elements may be substituted for heating elements 181 and 182, and a secondary amount of cooling provided as sensed by temperature sensor 183.

Furthermore, while the ACD and saline containers 43 and 44 have been shown as discrete containers, it will be appreciated that with appropriate modifications to fluid circuit 129, such as the provision of valves inline with the containers, these containers as well as containers for any other fluid, stored or collected in a procedure, could be formed directly within the fluid circuit, with a construction similar to that of chamber 150 of filter element 141.

Also, while the filter element 141 has been shown as formed within the fluid circuit, it will be appreciated that if desired this element can be provided as a discrete element, apart from the fluid circuit. Connections to the element would then be made by tubing segments, as with containers 43 and 44.

By reason of the compact fluid circuit made possible by the integrated housing and fluid circuit, fluid connections between components of the processing system of the invention are short and direct, so that, in the case of plasmapheresis, a minimal quantity of blood is removed at any one time from the donor during processing. This minimizes trauma to the donor.

Furthermore, by reason of the pumping and valving connections to the processor being automatically established upon installation of the system housing in associated processor apparatus, set-up time is minimized to the benefit of both the operator and the donor in the plasmapheresis procedure illustrated.

In contrast to conventional peristaltic pumps, the pumping and valving functions of the fluid circuit of the invention provide directly controlled, self-limiting pressure to cellular components passing through the circuit, thereby reducing the possibility of damage or lysing to these components from excessive pumping pressures in the event of an obstruction in the fluid circuit. Furthermore, the stress experienced by cellular components as they pass through the flow circuitry is significantly reduced in comparison to those experienced by peristaltic pumps and solenoid valve cutoffs. The circuit is thus very well suited for blood processing operations.

## Claims

1. A modular fliud processing system including at least one fluid container, a fluid-impermeable first member (36) provided wiht a generally outwardly depressed portion (70), a fluid-impermeable sheet member (71) overlaying the first member and forming in conjuction with the depressed portion a fluid circuit in fluid communication with the at least one fluid container (30, 43, 44) the fluid circuit including a pump element (130—137) operable in response to selectively, varying pressure on the sheet member to urge fluid through the circuit, and means (73 to 77) for connecting the fluid circuit to an element external to the module, charactrised in that the fluid processing system comprises a housing (22) having a base (36), side wall (32—35) upstanding from the base to define an open end and a space within the housing, and a removable cover (37) closing the open end, the base defining the first member (36), the sheet member being accomodated in the housing and being more flexible than the base, and the fluid container being accommodated in said space.

2. A system according to Claim 1 wherein said pump element (130—134) includes a fluid displacement chamber (a) formed inline in said fluid circuit, first and second valve stops (b and c) formed upstrevam and downstream of said displacement chamber in said fluid circuit, said flexible sheet member (71) being displaceable against said first and second valve stops and into said displacement chamber in response to the applied pressure to selectively urge fluid through said fluid circuit.

3. A syvstem according to Claim 1 or 2 including a filter (141) within the housing (22) and connected to the fluid circuit.

4. A system according to Claim 3 wherein said filter (141) is suitable for separating a blood fraction from whole blood.

5. A system according to Claim 4, wherein said fluid circuit includes means (27) for receiving whole blood from a donor and means (28) for returning component-deficient whole blood to the donor.

6. A system according to any preceding claim wherein the base (36) includes alignment means (89) for maintaining the base in operative alignment with an actuator (24) for actuating said pump elements.

7. A fluid system according to any preceding claim, wherein the cover (137) is peelably removable from the housing (22).

8. A fluid system according to any preceding claim, wherein the side walls include an access aperture (40) to facilitate application of pressure to said sheet member.

9. A fluid system according to any preceding claim, wherein the container (30, 43, 44) is connected in fluid communication with said fluid circuit, and removable from the housing (22) through said open end.

**Patentansprüche**

1. Ein Modul-Fluidprozeßsystem mit mindestens einem Fluidbehälter, einem fluidundurchlässigen ersten Element (36) mit einem im allgemeinen nach außen gewölbten Teil (70), einer fluidundurchlässigen Folie (71), die über dem ersten Element liegt und zusammen mit dem gewölbten Teil einen Fluidkreis in Fluidverbindung mit dem mindestens einen Fluidbehälter (30, 43, 44) bildet, wobei der Fluidkreis ein Pumpenelement (130—137) umfaßt, welches in Abhängigkeit von einem selektiven, variierenden Druck auf die Folie arbeitet, um Fluid durch den Kreis zu drängen, und mit Mitteln (73—77) zum Anschluß des Fluidkreises an ein Element außerhalb des Moduls, dadurch gekennzeichnet, daß das Fluidprozeßsystem ein Gehäuse (22) mit einem Boden (36), vom Boden abstehenden Seitenwänden (32—35) unter Definition einer offenen Seite und eines Raumes innerhalb des Gehäuses, und mit einer entfernbaren Abdeckung (37) zum Verschließen der offenen Seite aufweist, wobei der Boden das erste Element (36) definiert, die Folie im Gehäuse angeordnet und flexibler als der Boden ist, und wobei der Fluidbehälter sich in dem genannten Raum befindet.

2. System nach Anspruch 1, wobei das Pumpenelement (130—134) eine Fluidverlagerungskammer (a), die im genannten Fluidkreis in Reihe ausgebildet ist, und erste und zweite Ventilanschläge (b und c) aufweist, die stromaufwärts und stromabwärts von der Verlagerungskammer im Fluidkreis ausgebildet sind. wobei die flexible Folie (71) gegen die genannten ersten und zweiten Ventilanschläge und in die Verlagerungskammer bewegbar ist in Abhängigkeit vom angelegten Druck, um das Fluid durch den Fluidkreis selektiv zu drängen.

3. System nach Anspruch 1 oder 2, mit einem Filter (141) innerhalb des Gehäuses (22) und am Fluidkreis angeschlossen.

4. System nach Anspruch 3, wobei das genannte Filter (141) geeignet ist zur Trennung einer Blutfraktion vom Vollblut.

5. System nach Anspruch 4, wobei der Fluidkreis Mittel (27) zur Aufnahme von Vollblut von einem Spender und Mittel (28) zur Rückführung von von Komponenten befreitem Vollblut zum Spender aufweist.

6. System nach einem der vorangehenden Ansprüche, wobei der Boden (36) Fluchtungsmittel (89) zur Aufrechterhaltung des Bodens in operativer Fluchtung mit einem Betätigungsorgan (24) zur Betätigung der pumpenelemente aufweist.

7. Fluidsystem nach einem der vorangehenden Ansprüche, wobei die Abdeckung (137) vom Gehäuse (22) durch Abziehen entfernbar ist.

8. Fluidsystem nach einem der vorangehenden Ansprüche, wobei die Seitenwände eine Zugangsöffnung (40) aufweisen, um das Anlegen von Druck an die erwähnte Folie zu ermöglichen.

9. Fluidsystem nach einem der vorangehenden Ansprüche, wobei der Behälter (30, 43, 44) in Fluidverbindung mit dem genannten Fluidkreis verbunden, und vom Gehäuse (22) durch das erwähnte offene Seite entfernbar ist.

**Revendications**

1. Système modulaire de traitement de fluide comprenant au moins un récipient de fluide, un premier élément (36) imperméable aux fluides comportant une partie (70) généralement en creux vers l'extérieur, une feuille (71) imperméable aux fluides, superposée au premier élément et définissant en combinaison avec la partie en creux un circuit de fluide en communication de fluide avec ledit au moins récipient de fluide (30, 43, 44), le circuit de fluide comprenant un élément de pompe (130—137) qui agit en réponse à une pression sélectivement variable sur la feuille de manière à déplacer le fluide à travers le circuit, et des moyens (73à77) pour connecter le circuit de fluide à un élément extérieur au module, caractérisé en ce que le système de traitement de fluide comprend un boîtier (22) comportant une base (36), des parois latérales (32—35) verticales à la base pour définir une extrémité ouverte et un espace à l'intérieur du boîtier, et un couvercle amovible (37) fermant l'extrémité ouverte, la base définissant le premier élément (36), la feuille étant logée dans le boîtier et étant plus flexible que la base, et le récipient de fluide étant logé dans ledit espace.

2. Système suivant la revendication 1, dans lequel ledit élément de pompe (130—134) comprend une chambre (a) de déplacement de fluide formée en ligne dans ledit circuit de fluide, un premier et un deuxième sièges de vanne (b et c) formés en amont et en aval de ladite chambre de déplacement dans ledit circuit de fluide, ladite feuille flexible (71) étant déplaçable contre lesdits premier et deuxième sièges de vanne et dans ladite chambre de déplacement en réponse à la pression appliquée, pour déplacer sélectivement le fluide à travers ledit circuit de fluide.

3. Système suivant la revendication 1 ou 2, comprenant un filtre (141) placé dans le boîtier (22) et connecté au circuit de fluide.

4. Système suivant la revendication 3, dans lequel ledit filtre (141) est apte à séparer une fraction de sang du sang entier.

5. Système suivant la revendication 4, dans lequel ledit circuit de fluide comprend des moyens (27) pour recevoir du sang entier d'un donneur et des moyens (28) pour renvoyer le sang entier appauvri en composants au donneur.

6. Système suivant l'une quelconque des revendications précédentes, dans lequel la base (36) comprend des moyens d'alignement (89) pour maintenir la base en alignement opérationnel avec un actionneur (24) pour actionner lesdits éléments de pompe.

7. Système de fluide suivant l'une quelconque des revendications précédentes, dans lequel le couvercle (37) peut être enlevé du boîtier (22) par décollement.

8. Système de fluide suivant l'une quelconque des revendications précédentes, dans lequel les parois latérales comportent une ouverture d'accès (40) pour faciliter l'application de pression à ladite feuille.

9. Système de fluide suivant l'une quelconque des revendications précédentes, dans lequel le récipient (30, 43, 44) est connecté en communication de fluide avec ledit circuit de fluide et peut être enlevé du boîtier (22) par ladite extrémité ouverte.

FIG. 1

FIG. 4

1

FIG. 2

FIG. 3

SALINE

ACD

PLASMA

FIG. 5

# FIG. 6

FIG. 7

FIG.8

SALINE

A C D

# FIG.9

93  81  93

183

157

93  91  90  92  93

# FIG.10

108  102  100  108

94
167

103  103d
103c
103b
103a
101

163
164

183

123

106d  104d
106  106c  104  104a
104b
106b
106a  104c

107  105

162

108  170  108

112

111  110

EP 0 129 554 B1

FIG. 11

FIG. 12

EP 0 129 554 B1

FIG.13

FIG.14

FIG.15

8

FIG. 17

FIG. 22

FIG. 18

FIG. 23

FIG. 16

FIG. 24

EP 0 129 554 B1

# FIG.19A
## AT REST

# FIG.19B
## FILL STROKE

# FIG.19C
## PUMP STROKE

10

# FIG. 20A
## AT REST

# FIG. 20B
## FILL STROKE

# FIG. 20c
## PUMP STROKE

FIG. 21A
AT REST

FIG. 21B
FILL STROKE

FIG. 21C
PUMP STROKE

FIG. 21D
FILL STROKE

FIG. 21E
PUMP STROKE